# EUROPEAN PATENT SPECIFICATION

(11) **EP 0 435 859 B1**
(45) Date of publication and mention of the grant of the patent: **20.04.1994**
(21) Application number: 91200321.7
(22) Date of filing: 28.05.1987
(51) Int. Cl.: A61M 5/14, A61M 5/36

(54) **Administration set adapted for adding a beneficial agent and with self-priming ability**
Selbstansaugendes Zufuhrsystem zur Verabreichung eines Heilmittels
Système d'administration auto-amorçante adapté pour l'addition d'un remède

(30) Priority: 29.05.1986 US 868827
(43) Date of publication of application: 03.07.1991
(62) Divisional of application: 87903792.7
(73) Proprietor: BAXTER INTERNATIONAL INC., Deerfield, IL 60015 (US)
(72) Inventor: Zdeb, Brian D., Round Lake Park, Illinois 60073 (US); Slater, Glen L., Ingleside, Illinois 60041 (US)
(74) Representative: MacGregor, Gordon

(56) References cited:
- EP-A- 0 146 310
- EP-A- 0 162 531
- EP-A- 0 179 745

## Description

### Technical Field of the Invention

The present invention is related to the delivery of a beneficial agent to a patient and is more particularly directed to the passive delivery of the beneficial agent to the intravenous system of a patient in a safe and effective manner.

### Background of the Invention

Many drugs are mixed with a diluent before being delivered intravenously to a patient. The diluent may be, for example, a dextrose solution, a saline solution or even water. Many such drugs are supplied in powder form and packaged in glass vials or ampules. Other drugs, such as some used in chemotherapy, are packaged in glass vials or ampules in a liquid state.

Powdered drugs may be reconstituted in a well known manner, utilizing a syringe which is used to inject liquid into the vial for mixing, the syringe eventually withdrawing the mixed solution from the vial. When a drug must be diluted before delivery to a patient the drug is often injected into a container of diluent after it is reconstituted, where the container may be connected to an administration set for delivery to a patient. More specifically, the diluent is often packaged in glass bottles, or flexible plastic containers such as are sold under the names MINI-BAG™ AND VIAFLEX® by Travenol Laboratories, Inc. of Deerfield, Illinois. These containers have administration ports for connection to an administration set which delivers the container contents from the container to the patient. The drug is typically added to the container through an injection site on the container.

Drugs may be packaged separately from the diluent for various reasons. One of the most important reasons is that many drugs do not retain their chemical and physical stability when mixed with a diluent and thus cannot be stored for any substantial period of time. Also, drugs are often packaged separately from the diluent because many firms which manufacture drugs are not engaged in the business of providing medical fluids in containers for intravenous delivery, and vice versa.

Therefore, a doctor, nurse, pharmacist or other medical personnel must mix the drug and diluent. This presents a number of problems. The reconstitution procedure is time consuming and requires aseptic technique. The operator must provide the proper diluent and a syringe before beginning. Often the powdered drug is "caked" at the bottom of the vial. Thus, when liquid is injected into the vial from a syringe the surface area of contact between the liquid and the powdered drug may be quite small initially, thus making the mixing procedure even more time consuming. Because of the limited vial volume, the increasing drug concentration in the diluent makes it harder to finish the reconstitution process. The operator may attempt to solve this by repeatedly injecting solution into the vial, mixing and withdrawing the solution but this makes necessary additional injections and movement of the syringe which increase the likelihood of contamination. Also, it is sometimes difficult to get all of the drug and/or liquid out of the vial, thus increasing the time required to perform the reconstitution procedure.

The reconstitution procedure should be performed under preferably sterile conditions. In addition to such a requirement making the operator justifiably more cautious and consuming more time, sterile conditions are often hard to maintain. In some instances, a laminar flow hood may be required under which the reconstitution procedure is performed.

Some drugs, such as some chemotherapy drugs, are toxic. Exposure of the operator to the drugs during reconstitution may be dangerous, especially if the operator works with such drugs on a daily basis and is repeatedly exposed to them.

A further problem is that the reconstitution procedure provides a source of confusion as to which container contains which drug. The diluent container should be marked with the drug with which it has been injected and the name of the patient to whom it should be delivered.

After a drug is reconstituted and withdrawn into a syringe barrel, the drug may in some instances be injected immediately into the intravenous system of a patient. More typically however, the reconstituted drug is injected from the syringe into a larger container of solution as discussed above, for connection to an intravenous administration set. This is because often the drug reconstituted in the syringe is still at a concentration so high as to cause local toxicity in the veins of a patient near the injection site where the needle pierces the skin. This may create severe vein irritation which may be medically harmful. Additionally, even though the proper dose of medication is in the syringe, immediate injection into the patient's blood stream may create a condition of systemic toxicity wherein the level of drug concentration in the patient's entire blood stream is dangerously high. Yet another reason for not making the injection from the syringe directly into the patient is that it creates an additional injection site into the patient, which may be painful for the patient and provides another opportunity for infection.

For these reasons, the reconstituted drug is more typically injected into a diluent container.

A patient may typically be administered a dextrose or saline solution from a large volume parenteral container, for example, such as a one liter container, delivered through an administration set such as a CONTINU-FLO® administration set sold by Travenol Laboratories. If the reconstituted drug were injected into the large volume parenteral container, delivery of the drug would usually be delivered over too long a time period. Often, these large volume fluids are delivered at very slow flow rates.

More typically, the reconstituted drug is injected into a small volume parenteral container, such as a fifty milliliter container sold by Travenol Laboratories. This MINIBAG™ container is hung at a higher elevation than the large volume parenteral container and is connected by a secondary administration set to an injection site on the primary administration set. Because it is maintained at a higher elevation, the reconstituted drug in the small volume container is delivered, after which fluid from the large volume container begins to flow once more. By utilizing a small volume container connected to an administration set for delivery of the drug or other beneficial agent instead of a direct syringe injection, the drug is delivered over a preferred time period that tends to minimize negative side effects.

A closed reconstitution delivery system is disclosed in U.S. Patent Nos. 4,410,321; 4,411,662; 4,432,755; and 4,458,733. As shown therein, a container includes a drug and a diluent in separate compartments which are reconstituted in a closed system before the drug is delivered to the patient. Typically, the container is connected to an administration set which is connected at its other end to the primary administration set, such as with the small volume parenteral container described above. The container shown in these patents solves many of the problems associated with syringe reconstitution. The product does however necessitate a series of reconstitution steps which must be performed by the nurse or other operator prior to delivering the fluid from the container.

Delivery of a drug or other beneficial agent in a manner not requiring reconstitution steps by an operator is shown in U.S. Patent Nos. 4,424,056; 4,432,756; 4,439,183; 4,474,574; 4,479,793; 4,479,794; 4,525,162, and 4,548,599 and Canadian Patent No. 1,173,795, As disclosed in those patents, a parenteral delivery system is disclosed which has a formulation chamber therein for administering a beneficial agent such as a drug. The system is advantageous in that it provides for reconstitution of the drug by fluid flowing from a large volume parenteral container for example. through the administration set containing the formulation chamber with the drug therein. The system intends to eliminate the need for the time consuming reconstitution procedure described above and appears to eliminate the problems associated with the reconstitution procedure.

Another passive reconstitution system is disclosed in European Patent Application No. 0059694.

Still another device for delivering a drug "in-line", i.e., in the administration set, is disclosed in U.S. Patent No.4,534,757. The device holds the drug and includes a section through which the liquid passes in a direction substantially opposite to the general direction in which liquid flows to the patient.

Yet another system which attempts to provide for drug reconstitution in-line without manual reconstitution by a nurse or other operator is shown in U.S. Patent No. 4,465,471. That patent discloses constructions for a receptacle in the administration set itself. A separate cartridge containing the drug to be reconstituted and delivered to the patient is plugged into the receptacle. As liquid enters the cartridge for reconstitution of the drug and subsequent delivery out of the cartridge and receptacle and into the patient, some or most fluid continues to flow through the administration set, bypassing the cartridge entirely.

European Patent No. 0146310 Lilly and Co. is directed to a system for drug reconstitution including an intravenous administration set and a drug vial and utilizes the vial vacuum to reconstitute the drug. The use of a vial whose contents are under a vacuum avoids air from being forced through the system and into the patient.

U.S. Patent No. 4,534,758. discloses a relatively complex drug delivery apparatus with various valves. When liquid from a container is delivered to the drug vial, the vial is to be agitated for a time sufficient to suspend the previously dry medicine.

U.S. Patent No. 4,581,014 discloses a selector valve for delivering a previously reconstituted drug from a drug vial through an intravenous administration set to a patient.

All the publications described above are directed to solutions to the time consuming reconstitution procedure and/or its associated problems, such as delivery of the solution to a patient. In most of the offered solutions, delivery of the drug is intended to be passive, i.e., once the drug is placed into the administration set, manual reconstitution steps are not required.

Still another common feature of many of the attempted solutions disclosed in these publications is that delivery of the drug is intended to be able to be made in a manner which is essentially independent of the fluid flow rate through the administration set and into the patient. Stated differently, some of the systems are designed to deliver a certain dosage of drug in a preselected time period, within a broad range of fluid flow rates. Delivery of a drug independent of flow rate is desirable because it ensures that the necessary dosage will be delivered within a therapeutically acceptable time period, which may be typically about twenty to thirty minutes, although this time period may vary depending upon the drug and dosage.

By making delivery of the drug or other beneficial agent independent of the flow rate, the system ensures that the drug will not be delivered too quickly should the flow rate be set too high by the nurse or other operator, thereby preventing the problem of systemic toxicity discussed above.

Some of the documents, such as U.S. Patent Nos. 4,424,056; 4,479,793; and 4,479,794, are also directed to systems having a beneficial agent placed "in-line" in an administration set for mixing of the agent and delivery to a patient, wherein the delivery of the agent may be made in a given volume of fluid. Also, a valve controlling fluid flow may be manually operated to deliver the agent in a manner which can be made dependent upon fluid flow.

At least the automatic reconstitution type systems discussed above, (i.e., those not requiring a separate agitation or mixing step), suffer from the possibility of creating a concentration of beneficial agent in the fluid being delivered to the patient which is too high at low flow rates. This results in local toxicity to the patient near the point of introduction into the body. The problem is solved by the invention disclosed in EP-A-0203186 No. Further solutions to the problems of passively mixing and delivering a beneficial agent to a patient are disclosed in EP-A-0203162 which discloses certain housing constructions for delivering the beneficial agent to the patient. Typically, the housing includes a receptacle which is placed in-line in a medical liquid administration set and a separate cartridge including the beneficial agent. The cartridge is plugged into the receptacle when it is desired to deliver the beneficial agent to the patient. Active reconstitution by a nurse or other operator is not required. Instead, once the cartridge is plugged into the receptacle, liquid flowing from the source of medical liquid through the administration set flows into the receptacle and the agent-containing cartridge, reconstituting the agent. The solution with agent therein flows out the receptacle, down the administration set to the patient's venous system.

It would be desirable to have an administration set adapted for the passive mixing and delivery of a beneficial agent to a patient, which does not require any communication with the external environment.

The present invention provides an administration set for the delivery of a medical liquid from a liquid source to a patient, wherein the administration set is adapted for receiving an added beneficial agent for delivery to that patient, comprising:
a fluid conduit including upstream connection means for connection to a source of medical fluid and downstream connection means for connection to a patient's venous system;
a receptacle mounted along said fluid conduit; a cartridge containing beneficial agent and being mountable on said receptacle for entraining beneficial agent in medical fluid flowing through the receptacle;
a chamber along said fluid conduit downstream of said receptacle, said chamber including
(i) an inlet for receiving fluid from the upstream fluid conduit, and
(ii) an outlet in fluid communication with the downstream fluid conduit, said chamber

having a cross-sectional area that is greater than the cross-sectional area of said fluid conduit, so that liquid entering said chamber inlet drops to said chamber outlet, there being in normal operation some retained air between said chamber inlet and the liquid disposed immediately above said chamber outlet, characterised in that the cartridge contains air which is forced downstream through the receptacle in use, and said chamber is designed by an air flash adapted to collect the air so as to prevent that air from passing downstream to a patient.

### Description of the Drawings

Fig. 1 is a perspective view of an administration set including the air flask and the receptacle;
Fig. 2 is an enlarged, fragmentary, cross-sectional view of the receptacle illustrated in Figure 1;
Fig. 3 is a fragmentary, enlarged, cross-sectional view of the air flask illustrated in Fig. 1;
Fig. 4 is a side elevational view of a cartridge for beneficial agent to be used with the administration set of Figure 1, illustrating the cartridge chamber in the first position;
Fig. 5 is a side elevational view as in Fig. 4, with the cartridge chamber having been slidably moved to its second position;
Fig. 6 is a fragmentary cross-sectional view of the closure for the cartridge chamber;
Fig. 7 is a fragmentary perspective view of the administration set prior to mounting the cartridge upon the receptacle;
Fig. 8 is a view similar to Fig. 7, illustrating the cartridge mounted upon the receptacle;
Fig. 9 is a fragmentary, cross-sectional view of the administration set, illustrating the receptacle, the cartridge and the air flask in fluid communication;
Fig. 9A is a cross-sectional view of another embodiment of the cartridge, with the cartridge chamber in the first position;
Fig. 9B is a cross-sectional view of the cartridge of Fig. 9A, with the cartridge chamber in the second position.

### Detailed Description

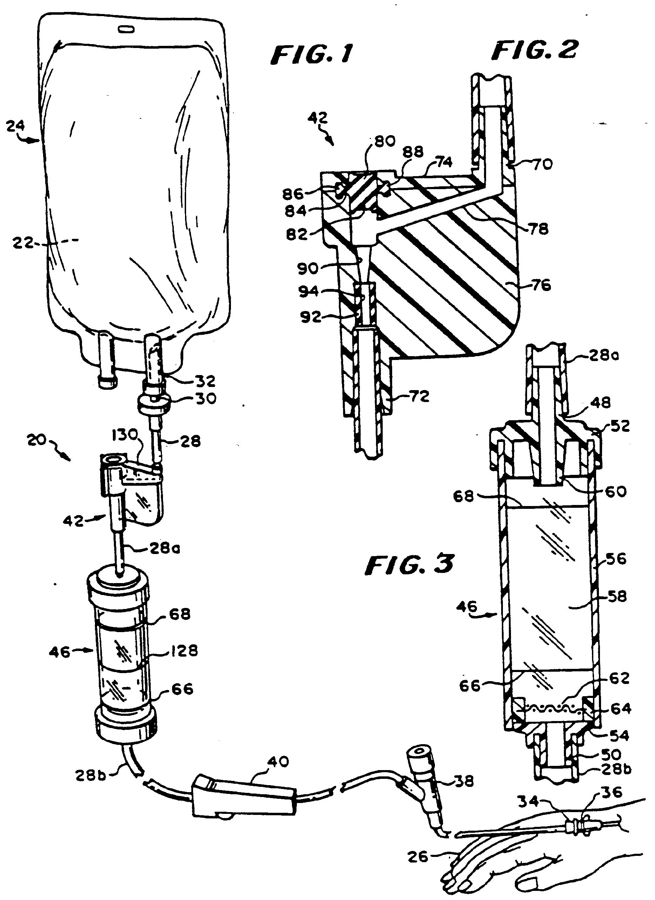

Referring to Fig. 1, there is illustrated an administration set 20 for the delivery of a medical liquid, stored within a medical liquid source such as large volume parenteral container 24, to a patient 26. The administration set 20 includes a fluid conduit 28 made for example of flexible polyvinylchloride tubing. Upstream connection means such as a standard intravenous administration set spike 30 is mounted at the upstream end of the fluid conduit 28. The spike is adapted for piercing the membrane of the container administration port 32.

The fluid conduit 28 includes downstream connection means such as a Luer taper 34 mounted at the downstream end of the fluid conduit 28. The Luer taper 34 may be connected in accordance with standard technique to a venous catheter 36.

The administration set 20 may further include a standard pierceable injection site 38 for injecting a medical liquid by means of a needle through the injection site 38. The administration set 20 may further include flow rate control means such as a standard roller clamp 40 mounted about the flow conduit 28.

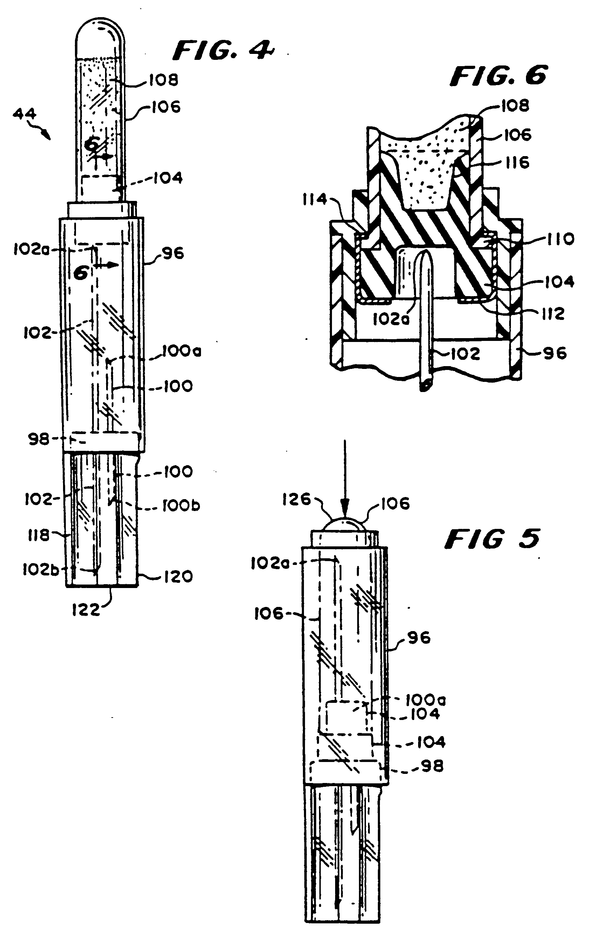

The administration set 20 further includes a unique receptacle 42 shown in greater detail in Fig. 2. The receptacle 42 is an improvement to the receptacle disclosed in W0-A-8603417. The receptacle 42 is mounted along the fluid conduit and is adapted for receiving a separate cartridge 44 containing beneficial agent, illustrated in Figs. 4 through 9 and 10. When the cartridge is mounted upon the receptacle, at least some, and preferably all liquid from the medical liquid source container 24 that flows through the fluid conduit 28 into the receptacle 42 also flows through the cartridge 44 before passing downstream out of the receptacle to the patient.

Downstream of the receptacle 42 is an air chamber 46, illustrated in Figs. 1, 3, 7, 8 and 9. As will be explained in greater detail below, the air flask 46 permits automatic priming of the cartridge 44 upon mounting of the cartridge on the receptacle 42 of the administration set 20. The air flask 46 absorbs the air disposed within the cartridge 44 and prevents that air from passing downstream to the patient.

Referring to Fig. 3, the air flask 46 includes an inlet 48 mounted to and receiving fluid from upstream fluid conduit 28a. The air flask 46 includes an outlet 50 mounted to and transferring liquid to downstream fluid conduit 28b. The inlet and outlet may be mounted to the fluid conduit 28 by means of interference fit, solvent bonding etc. The air flask 46 is mounted downstream of the receptacle 42.

In the preferred embodiment the air flask 42 includes inlet and outlet end caps 52, 54 respectively between which is mounted a cylindrical sidewall 56 of preferably optically transparent, flexible material such as polyvinyl chloride . The sidewall 56 and the end caps 52, 54 define an air chamber 58 having a cross-sectional diameter that is greater than the internal diameter of the fluid conduit 28, so that liquid entering the air chamber 58 from the drop forming orifice 60 adjacent the inlet 48 falls toward the outlet 50. The air flask 46 thus provides a collection reservoir for air within the administration set 20.

The air flask 46 further includes particulate matter barrier means such as a particulate matter screen 62 mounted within a plastic ring 64 near the outlet 50. The particulate matter barrier may in fact be a sterilizing filter having a nominal pore size of about 0.2 micron. The nominal pore size may be much larger, such as a gross particulate matter barrier having a nominal pore size of about 20 microns. In the preferred embodiment the nominal pore size is about 10 microns. The screen may be a nylon mesh material such as supplied by Filter Tek of Hebron, Illinois. The particulate matter barrier 62 is mounted transverse to the fluid path such that all liquid passing through the air flask 46 must pass through the particulate matter barrier 62.

The particulate matter barrier 62 need not be disposed within the air flask 46 but the barrier should be mounted downstream of the receptacle 42 so that all liquid that exits the inserted cartridge 44 will pass through the particulate matter barrier. Also, it is possible to construct the receptacle 42, the air flask 46 and the particulate matter barrier 62 as a single unit rather than as separated by upstream fluid conduit 28a for example.

In the preferred embodiment, the air flask 46 includes a minimum liquid level indicator 66 and a maximum liquid level indicator 68 which may for example comprise lines around the periphery of the air flask 46. The liquid level in the air flask 46 should preferably be somewhere between the minimum and maximum liquid level indicators 66, 68 immediately before insertion of the cartridge 44 within the receptacle 42.

The improved receptacle 42 includes a receptacle inlet 70 and a receptacle outlet 72 connected to the fluid conduit 28. The air flask 46 is disposed downstream of the receptacle outlet 72.

The receptacle 42 includes upper and lower fitments 74, 76 respectively. The upper fitment 74 includes the inlet. The lower fitment 76 includes the outlet 72 and a fluid receiving segment 78 having an upstream end in fluid communication with the inlet 70 and a downstream end in fluid communication with the outlet 72.

Pierceable situs 80 is mounted within the receptacle 42, trapped between the upper and lower fitments 74, 76. The pierceable situs 80 includes a pierceable main body portion 82 and a ring-like extension 84 extending about the periphery of the main body portion 82. The ring-like extension 84 further includes an enlarged outer periphery 86.

Together, the upper and lower fitments 74, 76 define an annular channel 88 substantially corresponding to and receiving the ring-like extension 84 including the enlarged periphery 86 thereof, such that the upper and lower fitments trap the pierceable situs 80 therebetween in secure fashion. The situs 80 may not be removed without destruction of the receptacle 42. The upper and lower fitments 74, 76 may be bonded together by adhesive, ultrasonic sealing etc. It is important that the situs be securely maintained within the receptacle because a plurality of cartridges 44, each having two piercing cannulas, may be repeatedly inserted and withdrawn from the situs during the useful life of the receptacle 42 and administration set 20. The fluid receiving segment 78 includes a tapered portion 90 below and generally coaxial with the pierceable situs 80. The tapered portion 90 serves as a needle guide into the resilient bushing 92.

The resilient bushing is preferably made of an elastomer such as polyisoprene. The resilient bushing 92 defines a narrow through-bore 94. The resilient bushing is juxtaposed relative to the pierceable situs 80 so that the through-bore 94 is substantially coaxial with the tapered portion 90.

Turning now to Figs. 4 through 9 and 10 there is shown the cartridge 44 for introducing a drug or other beneficial agent into the fluid conduit 28 at the receptacle 42, for delivery of the agent to a patient.

The cartridge 44 includes a rigid cylinder 96 and a base plate 98 mounted across the rigid cylinder 96. First and second hollow cannulas 100, 102 respectively are mounted through the base plate 98 and extend within the rigid cylinder 96 on at least one side of the base plate in a direction substantially parallel to and inside the rigid cylinder 96. Each of the hollow cannulas 100, 102 extend on both sides of the base plate. The first hollow cannula 100 includes a pointed first end 100a adapted for piercing a pierceable stopper 104. The first hollow cannula 100 also includes a pointed second end 100b opposite the pointed first end 100a. Similarly, the second hollow cannula 102 includes a pointed first end 102a adapted for piercing the pierceable stopper 104. The second hollow cannula 102 also includes a second pointed end 102b opposite the pointed end 102a. The second hollow cannula 102 extends further from the base plate on both sides thereof than the first hollow cannula 100.

The cartridge 44 further includes a tubular chamber 106 containing a beneficial agent 108 such as a dry powdered drug, although the agent may also be a liquid. The pierceable stopper 104 or other closure means previously referred to above closes the tubular chamber 106.

Referring to Figure 6, the pierceable stopper 104 is mounted within the mouth 110 of the tubular chamber 106. The rubber stopper 104 may be secured within the tubular chamber 106 by means of a metal band 112 about the periphery of the mouth 110 and the rubber stopper 104, in a manner similar to the securement of a stopper in a standard drug vial. The tubular chamber 106 is slidably mounted within the rigid cylinder 96 such that the rubber stopper 104 faces the base plate 98. The tubular chamber 106 is kept from total disengagement from the cylinder 96 by means of a lip 114 extending from the rigid cylinder 96. The lip 114 engages the stopper 104 and metal band 112 assembly that extends outwardly from the sidewall of the tubular chamber 106, as illustrated in Fig. 6. The pierceable stopper 104 may include a cone defining volume 116 facing the interior of the chamber 106. In place of the pierceable stopper, other pierceable closure means may be provided.

When the cartridge 44 is in a first position illustrated in Figs. 4, 6 and 7 for example, the rubber stopper 104 has not been pierced through by either the first or second hollow cannulas 100, 102. In the preferred embodiment, the pierceable stopper 104 remains spaced from the first and second cannula 100, 102 when the tubular cartridge 106 is in the first position.

The first and second hollow cannulas 100, 102 comprise flow path means. The shorter, first hollow cannula 100 provides an inlet path into the tubular chamber 106. The longer, second cannula 102 provides an outlet path out of the chamber. The flow path means forms part of the adapter means, including the rigid cylinder, mounted about the chamber and adapted for mounting the cartridge 44 upon the receptacle 42. The adapter slides relative to the chamber 106. As will be seen later in other embodiments, the hollow cannulas 100, 102 may be slidable within the rigid cylinder. Stated differently, the tubular chamber and the adapter flow path means are selectively slidable relative to each other.

The adapter means may further include keyway means extending on the side of the base plate opposite of the chamber 106 and substantially coaxial therewith. The keyway means may include a relatively rigid keyway wall 118 including a keyway slot 120 for fitting over the receptacle 42. The keyway wall 118 may also include one or more longitudinal defined channels 122 for engaging corresponding longitudinal keys 124 mounted about the exterior of the receptacle 42. The keyway means ensures proper engagement of the cartridge 44 with the associated receptacle 42, including the proper disposition of the first and second hollow cannulas 100, 102 within the receptacle 42.

The chamber 106 of the cartridge 44 is slidable from the first position shown in Fig. 4 for example to a second position illustrated in Fig. 5 obtained by pushing the chamber 106 down within the rigid cylinder 96 until the pierceable stopper 104 abuts the base plate 98, which serves as a stop. In this position, both the first and second cannulas 100, 102 have pierced the pierceable stopper 104, so that the pointed hollow ends 100a, 102a of the first and second cannulas 100, 102 are in communication with the chamber interior. The end 102a of the second hollow cannula is well within the tubular chamber, preferably near the top end 126 of the chamber 106. The pointed hollow end 100a of the first cannula 100 is preferably just within the tubular chamber 106, such as within the hollow conical portion 116 defined by the stopper 104.

In operation, before a beneficial agent 108 in the cartridge is delivered to the patient, the administration set 20 of the invention operates by providing an open fluid pathway between the medical liquid container 24 and the patient 26, as illustrated in Fig. 1. Liquid 22 flows from the container 24 through the administration port 32 and spike 30. The liquid flows through the fluid conduit 28 and through the receptacle 42, following the pathway through the receptacle inlet 70, fluid receiving segment 78, tapered portion 90, through-bore 94 and outlet 72, in that order. Liquid flows through the connecting conduit 28 and into the air flask 46 through the drop former 60. Air collects within the air flask 46 and liquid continues to flow downstream through the flask outlet 50 through the downstream conduit portion 28b and into the patient through the Luer connection 34 and venous catheter 36.

Before the administration set 20 is placed in communication with the patient 26, the fluid conduit 28 is primed, i.e., air is eliminated. This is performed in the known manner, by allowing liquid to flow through the set 20 before connection to the patient.

To raise the liquid level up to a level 128 within the flask 46 such that it is between the minimum and maximum indicator lines 66, 68, the air flask sidewall 56 may be squeezed and released such as with most drip chambers, in the standard manner.

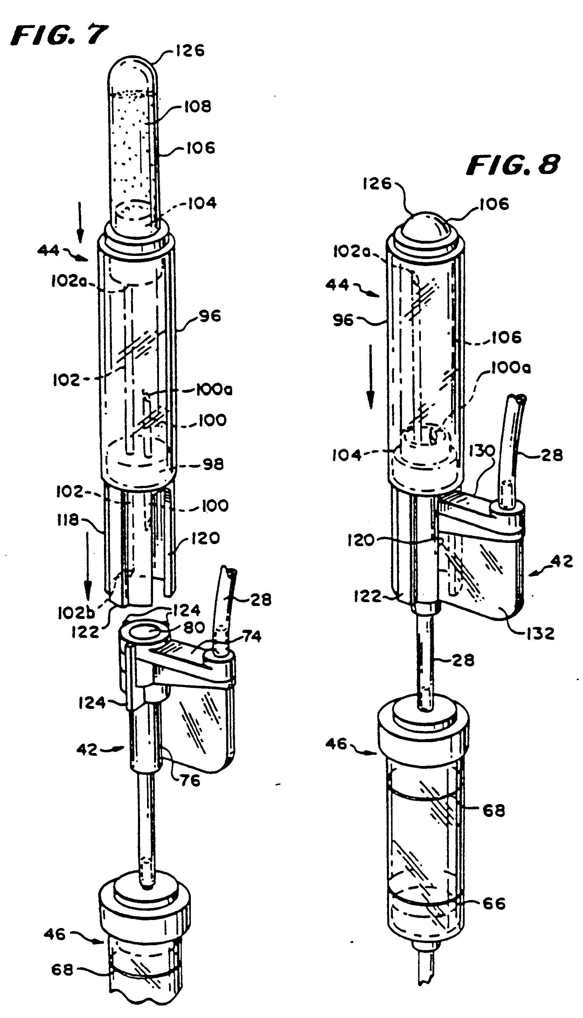

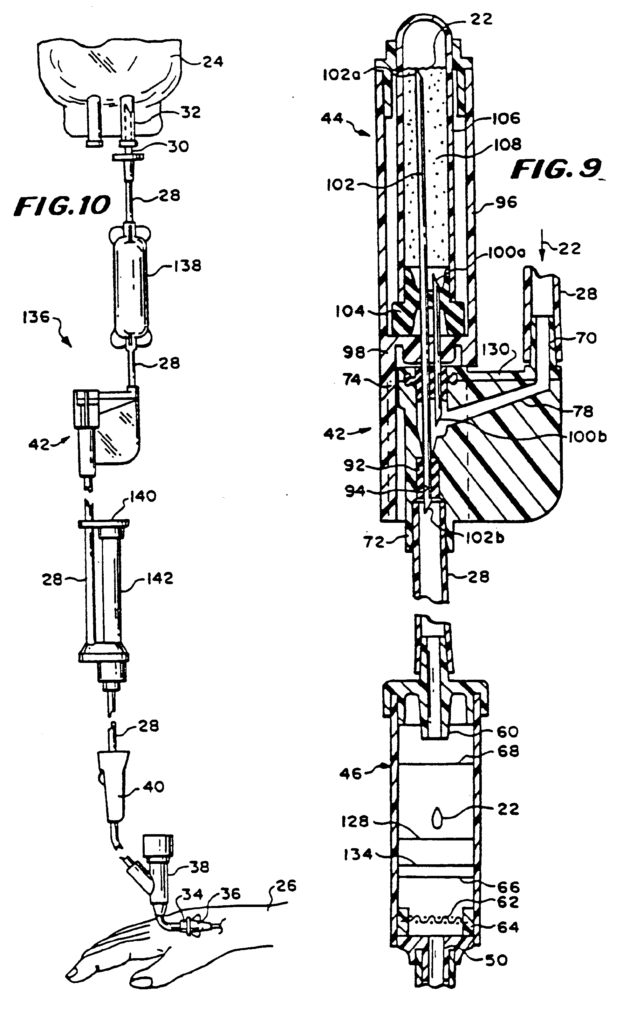

When it is desired to deliver a beneficial agent 108 such as a drug to the patient, the cartridge 44 having the beneficial agent 108 therein is mounted upon the receptacle 42. Fig. 7 illustrates the cartridge 44 and receptacle 42 before activation of the cartridge and before it is mounted on the receptacle.

The cartridge is provided to the nurse or the medical personnel as illustrated in Figs. 4 and 7, with the chamber 106 in the first position. The cartridge is activated simply by grasping the rigid cylinder 96 and pushing down on the top 126 of the chamber 106 with the thumb. This forces first the second cannula end 102a and then the first cannula end 100a through the pierceable stopper 104. The tubular chamber 106 is urged downwardly until further movement is limited by contact between the pierceable closure 104 and the base plate 98. This second position is illustrated in Fig. 5.

With the cartridge 44 now in the second position, the cartridge is then mounted upon the receptacle 42 as illustrated in Fig. 8. It is important that the first and second cannulas 100, 102 be disposed in a particular position within the receptacle 42. This is provided for by the keyway wall 118 having the keyway slot 120 therewithin, the slot 120 being guided over the bridge 130 of the upper fitment 74 on the receptacle 42; and is further provided for by the longitudinal defined channels 122 within the keyway wall 118, which fit over the plurality of longitudinal keys 124 mounted about the receptacle 42. In the preferred, illustrated embodiment the keyway wall 118 includes three defined channels 122 for mating with three longitudinal keys 124 on the receptacle.

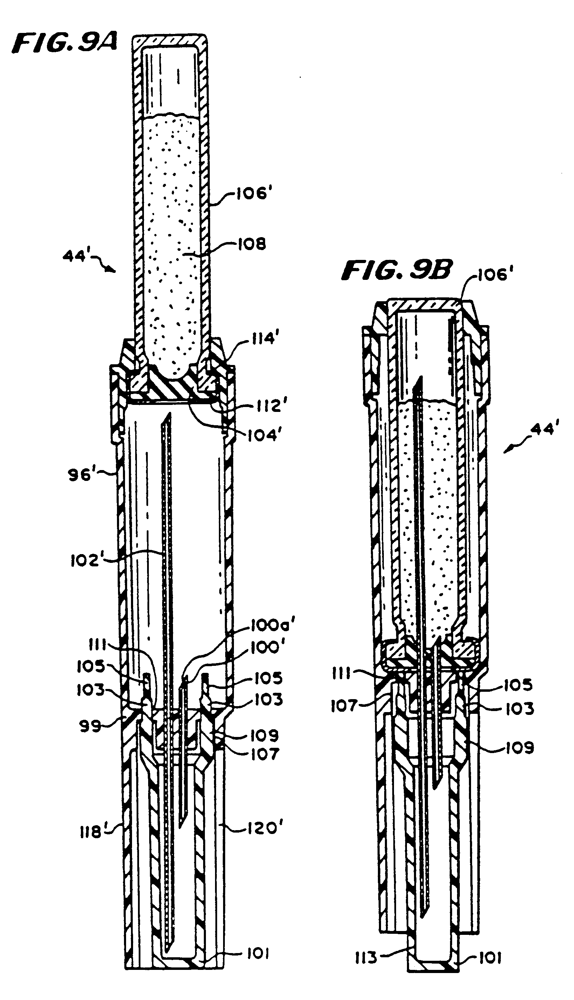

Referring to Fig. 9, the cartridge 44 is easily mounted upon the receptacle 42 in the manner shown in Fig. 8 by grasping the receptacle 42 at the handle 132 in one hand and the rigid cylinder 96 in the other hand and pushing the cartridge down so that the second end 102b of the second cannula and then the second end 100b of the shorter, first cannula both pierce the main body portion 82 of the pierceable situs 80. The cartridge 44 continues to be urged downwardly so that the second hollow cannula 102 enters the through-bore 94 and is liquid-sealingly engaged by the bushing 92 around the periphery of the second hollow cannula 102. Proper installation has occurred after the base plate 98 abuts the top fitment 74, limiting further downward movement of the cartridge 44.

Upon engagement of the cartridge 44 and receptacle 42 as illustrated in Fig. 9, liquid 22 flowing into the receptacle at inlet 70 flows through the fluid receiving segment 78. The resilient bushing 92 has sealed about the second hollow cannula 102, preventing liquid 22 from passing immediately downstream. Instead, the liquid 22 enters the second end 100b of the first cannula 100 and enters the tubular chamber 106 at the first end 100a of the cannula.

As liquid 102 rises within the chamber 106, residual air within the chamber is forced downstream through the second hollow cannula 102. The air enters the air flask 46 through the drop former 60 and collects within the flask 46. The initial liquid level 128 illustrated in Fig. 1 drops to a new level such as indicated by line 134. The liquid level 128 should be above the minimum liquid level indicator line before insertion of the cartridge 44 into the administration set 20 so that as air exits the cartridge 44, the liquid level within the air flask 46 will not drop to the flask outlet 50 where it could be trapped and forced downstream to the patient. The liquid level 134 after cartridge priming may be below the minimum liquid level 66, but if it is above the minimum line 66 before insertion of the cartridge 44, the liquid level 134 will never be as low as the outlet 50.

The maximum liquid level indicator 68 serves as a guide for the maximum liquid level so that liquid drops entering the air flask through the drop former 60 may still be counted in the manner of a standard drip chamber.

The liquid level within the tubular chamber 106 continues to rise until it reaches the hollow pointed end 102a of the second cannula 102, whereupon liquid 72 begins to exit the chamber 106 through the second cannula 102, downstream through the second end 102b and into the air flask 46 through the drop former 60. Liquid exiting the chamber 106 has an appropriate concentration of beneficial agent 108 mixed therewith for delivery to the patient. The upward liquid flow path created within the chamber 106 by the first and second cannulas 100, 102 creates a density gradient within the chamber 106 such that the concentration of drug within the liquid 22 exiting at cannula end 102a will not be so high as to create local toxicity to the patient. Local toxicity is a situation in which vein irritation can occur near the venous injection site when drug concentrations within the delivery liquid 22 are too high.

At typical liquid flow rates, the amount of drug delivered to the patient per unit time is generally independent of the flow rate. This means that at extremely high flow rates, the total amount of drug delivered to the patient per unit time will not be so high as to cause systemic toxicity to the patient. Stated differently, the patient will not have too much drug introduced into the body in too short a time period.

It is believed that at lower liquid flow rates the rate of drug delivered to the patient per unit time tends to become more dependent upon the liquid flow rate through the administration set 20. However, local toxicity to the patient will not occur. It is believed that the upper limit on the drug concentration within liquid 22 exiting the chamber 106 is limited to a safe maximum for two principle reasons. First, the density gradient created within the columnar tubular chamber 106 means that the concentration of liquid 22 at the point of entry into the second cannula 102 is the lowest of any elevation within the tubular chamber 106. Secondly, as the liquid flow rate through the administration set 20 decreases, which would ordinarily increase the risk of an unacceptably high drug concentration to the patient, the amount of mixing and liquid turbulance created within the chamber 106 also decreases, exaggerating the density gradient so that the difference in densities from the area of the stopper 104 to the first end 102a of the second cannula 102 becomes greater.

It is to be noted that the different liquid flow rates mentioned above are only possibilities; in the preferred manner of operation, the nurse or other medical personnel would set an acceptable flow rate with the flow rate control means (such as the roller clamp 40 or a peristaltic pump) and not adjust the liquid flow rate again, at least until after delivery of the beneficial agent 108.

The administration set 20, with the unique cartridge 44 and receptacle 42, are capable of delivering a therapeutically beneficial amount of a beneficial agent 108 within a therapeutically acceptable time period. For example, a one gram dose of ampicillin in the chamber 106 may be delivered in about thirty minutes at a liquid flow rate of 120 mls per hour.

In the preferred embodiment, the tubular chamber 106 has a volume of about 10 mls, and may include up to about 3 to 4 mls of air. The internal diameter of the tubular chamber is about 10.16 mm (0.4 inch). The height of the tubular chamber from the mouth 110 to the top 126 is about 50.8 mm (two inches). As described in WO-A-8603417, the hollow conical portion 116 of the pierceable stopper closure 104 is believed to assist in mixing, creating additional turbulance at the point of entry of liquid 22 at the first end 100a of the first cannula 100. The relatively long, narrow configuration of the chamber 106 is also believed to assist in mixing the beneficial agent 108 with the liquid 22. The liquid 22 may be a 5% dextrose solution for example.

It is to be noted that by varying the dimensions of the tubular chamber 106 the delivery profile for the beneficial agent 108 may be changed. For example, by enlarging the internal diameter of the tubular chamber, it will take longer to deliver the agent 108 within the chamber 106 to the patient 26. Similarly, lengthening the chamber 106 will also increase the delivery time if the second cannula 102 is also extended within the longer chamber 106.

## Claims

1. An administration set for the delivery of a medical liquid from a liquid source to a patient, wherein the administration set is adapted for receiving an added beneficial agent for delivery to that patient, comprising:
a fluid conduit (28) including upstream connection means (30) for connection to a source (24) of medical fluid and downstream connection means (34) for connection to a patient's venous system;
a receptacle (42) mounted along said fluid conduit; a cartridge (44) containing beneficial agent (108) and being mountable on said receptacle (42) for entraining beneficial agent in medical fluid flowing through the receptacle;
a chamber (58) along said fluid conduit (28) downstream of said receptacle (42), said chamber including
(i) an inlet (48) for receiving fluid from the upstream fluid conduit, and
(ii) an outlet (50) in fluid communication with the downstream fluid conduit,
said chamber having a cross-sectional area that is greater than the cross-sectional area of said fluid conduit (28), so that liquid entering said chamber inlet (48) drops to said chamber outlet (50), there being in normal operation some retained air between said chamber inlet (48) and the liquid disposed immediately above said chamber outlet (50),
characterised in that the cartridge (44) contains air which is forced downstream through the receptacle (42) in use, and said chamber (58) is defined by an air flask (46) adapted to collect the air so as to prevent that air from passing downstream to a patient.

2. An administration set according to Claim 1, wherein the air flask (46) includes inlet and outlet end caps (42,54) between which is mounted a cylindrical sidewall (56), the inlet end cap (52) being formed with a drop-forming orifice (60) adjacent the inlet (48).

3. An administration set according to Claim 1 or 2, including a particulate matter barrier (62) between the inlet (48) and the outlet (50) in the chamber (58).

4. An administration set according to Claim 3, wherein said barrier (62) is a filter of nominal pore size of 0.2 to 20 microns.

5. An administration set according to Claim 4, wherein said nominal pore size is 10 microns.

6. An administration set according to any preceding claim, including a minimum liquid level indicator (66) and a maximum liquid level indicator (68) for the air flask (46).

7. An administration set according to any preceding Claim, wherein the receptacle (42) and the air flask (46) are constructed as a single unit.

8. An administration set according to any preceding claim including liquid flow rate control means (40) downstream of the air flask (46).

9. An administration set according to any preceding claim including an injection site (38) mounted along the fluid conduit (28) downstream of the air flask (46).

10. An administration set according to any preceding claim, wherein the air flask (46) is externally manipulable to reduce the internal volume thereof.

## Patentansprüche

1. Verabreichungsset zur Abgabe einer medizinischen Flüssigkeit von einer Flüssigkeitsquelle an einen Patienten, wobei das Verabreichungsset so ausgebildet ist, daß es ein hinzugefügtes gesundheitsförderndes Mittel zur Abgabe an diesen Patienten aufnehmen kann, und folgendes aufweist:
- eine Fluidleitung (28), die eine aufstromseitige Verbindungseinrichtung (30) zur Verbindung mit einer Quelle (24) für medizinisches Fluid und eine abstromseitige Verbindungseinrichtung (34) zur Verbindung mit dem Venensystem eines Patienten aufweist;
- einen Behälter (42), der entlang der Fluidleitung angebracht ist;
- eine Patrone (44), die ein gesundheitsförderndes Mittel (108) enthält und an dem Behälter (42) anbringbar ist, um ein gesundheitsförderndes Mittel in dem medizinischem Fluid, das den Behälter durchströmt, mitzunehmen;
- eine Kammer (58) entlang der Fluidleitung (28) an der Abstromseite des Behälters (42), wobei die Kammer folgendes aufweist:
(i) einen Einlaß (48), um Fluid von der aufstromseitigen Fluidleitung aufzunehmen, und
(ii) einen Auslaß (50) in Fluidverbindung mit der abstromseitigen Fluidleitung,
- wobei die Kammer eine Querschnittsfläche hat, die größer als die Querschnittsfläche der Fluidleitung (28) ist, so daß in den Kammereinlaß (48) eintretende Flüssigkeit zu dem Kammerauslaß (50) tropft, wobei im Normalbetrieb etwas zurückgehaltene Luft zwischen dem Kammereinlaß (48) und der sich unmittelbar über dem Kammerauslaß (50) befindlichen Flüssigkeit ist,
dadurch gekennzeichnet,
daß die Patrone (44) Luft enthält, die im Gebrauch in Abstromrichtung durch den Behälter (42) gedrückt wird, und daß die Kammer (58) von einem Luftkolben (46) gebildet ist, der so ausgebildet ist, daß er die Luft sammelt, um diese Luft daran zu hindern, zur Abstromseite zu einem Patienten zu gelangen.

2. Verabreichungsset nach Anspruch 1,
wobei der Luftkolben (46) Einlaß- und Auslaßenkappen (42, 54) aufweist, zwischen denen eine zylindrische Seitenwand (56) angebracht ist, wobei die Einlaßendkappe (52) mit einer tropfenbildenden Öffnung (60), angrenzend an den Einlaß (48), ausgebildet ist.

3. Verabreichungsset nach Anspruch 1 oder 2,
das eine Sperre (62) für teilchenförmige Stoffe zwischen dem Einlaß (48) und dem Auslaß (50) in der Kammer (58) aufweist.

4. Verabreichungsset nach Anspruch 3,
wobei die Sperre (62) ein Filter mit einer Nennporengröße von 0,2 bis 20 µm ist.

5. Verabreichungsset nach Anspruch 4,
wobei die Nennporengröße 10 µm ist.

6. Verabreichungsset nach einem der vorhergehenden Ansprüche,
das eine Flüssigkeitsminimalpegelanzeige (66) und eine Flüssigkeitsmaximalpegelanzeige (68) für den Luftkolben (46) aufweist.

7. Verabreichungsset nach einem der vorhergehenden Ansprüche,
wobei der Behälter (42) und der Luftkolben (46) als eine einzige Einheit ausgebildet sind.

8. Verabreichungsset nach einem der vorhergehenden Ansprüche,
das eine Steuereinrichtung (40) für die Flüssigkeitsdurchflußrate an der Abstromseite des Luftkolbens (46) aufweist.

9. Verabreichungsset nach einem der vorhergehenden Ansprüche,
das eine Injektionsstelle (38) aufweist, die entlang der Fluidleitung (28) an der Abstromseite des Luftkolbens (46) angebracht ist.

10. Verabreichungsset nach einem der vorhergehenden Ansprüche,
wobei der Luftkolben (46) von außen manipulierbar ist, um sein Innenvolumen zu verringern.

## Revendications

1. Ensemble d'administration pour la distribution d'un liquide médical d'une source de liquide à un patient, dans lequel l'ensemble d'administration peut recevoir un agent thérapeutique ajouté pour distribution à ce patient, comprenant :
un conduit de fluide (28) comportant des moyens de connexion amont (30), pour connexion à une source (24) de fluide médical, et des moyens de connexion aval (34) pour connexion au système veineux d'un patient ;
un réceptacle (42) monté le long dudit conduit de fluide ;
une cartouche (44) contenant un agent thérapeutique (108) et étant montable sur ledit réceptacle (42), de manière à entraîner l'agent thérapeutique dans le fluide médical qui circule à travers le réceptacle ;
une chambre (58) montée le long dudit conduit de fluide (28) en aval dudit réceptacle (42), ladite chambre comportant
- une entrée (48) pour recevoir le fluide venant du conduit de fluide amont, et
- une sortie (50) en communication de fluide avec le conduit de fluide aval,
ladite chambre ayant une section transversale qui est plus grande que la section transversale dudit conduit de fluide (28), de sorte que le liquide qui entre à ladite entrée de chambre (48) s'égoutte vers ladite sortie de chambre (50) et, en fonctionnement normal, de l'air est retenu entre ladite entrée de chambre (48) et le liquide disposé immédiatement au-dessus de la dite sortie de chambre (50) ;
caractérisé en ce que la cartouche (44) contient de l'air qui est chassé vers l'aval à travers le réceptacle (42) en utilisation, et ladite chambre (58) est définie par une bouteille d'air (46) capable de collecter l'air de façon à empêcher cet air de passer en aval vers un patient.

2. Ensemble d'administration suivant la revendication 1, dans lequel la bouteille d'air (46) comprend des chapeaux d'extrémité d'entrée et de sortie (52,54) entre lesquels est montée une paroi latérale cylindrique (56), le chapeau d'extrémité d'entrée (52) comportant un orifice de formation de goutte (60) adjacent à l'entrée (48).

3. Ensemble d'administration suivant la revendication 1 ou 2, comprenant un dispositif d'arrêt des particules (62) entre l'entrée (48) et la sortie (50) de la chambre (58).

4. Ensemble d'administration suivant la revendication 3, dans lequel ledit dispositif d'arrêt (62) est un filtre ayant une dimension nominale de pore de 0,2 à 20 µm.

5. Ensemble d'administration suivant la revendication 4, dans lequel ladite dimension nominale de pore est de 10 µm.

6. Ensemble d'administration suivant une quelconque des revendications précédentes, comprenant un indicateur de niveau minimal de liquide (66) et un indicateur de niveau maximal de liquide (68) pour la bouteille d'air (46).

7. Ensemble d'administration suivant une quelconque des revendications précédentes, dans lequel le réceptacle (42) et la bouteille d'air (46) sont construits sous la forme d'un élément unique.

8. Ensemble d'administration suivant une quelconque des revendications précédentes, comprenant des moyens de réglage de débit de liquide (40) placés en aval de la bouteille d'air (46).

9. Ensemble d'administration suivant une quelconque des revendications précédentes, comprenant un site d'injection (38) monté le long du conduit de fluide (28) en aval de la bouteille d'air (46).

10. Ensemble d'administration suivant une quelconque des revendications précédentes, dans lequel la bouteille d'air (46) est manipulable extérieurement de manière à réduire son volume intérieur.
